## Europäisches Patentamt

## European Patent Office

## Office européen des brevets

(11) Publication number: **0 043 738**

**B1**

(12) **EUROPEAN PATENT SPECIFICATION**

(45) Date of publication of patent specification: **02.10.85**

(51) Int. Cl.⁴: **A 61 K 9/06, A 61 K 47/00**

(21) Application number: **81303128.3**

(22) Date of filing: **09.07.81**

(54) Penetrating topical pharmaceutical compositions.

(30) Priority: **09.07.80 US 167167**

(43) Date of publication of application:
**13.01.82 Bulletin 82/02**

(45) Publication of the grant of the patent:
**02.10.85 Bulletin 85/40**

(84) Designated Contracting States:
**BE CH DE FR GB IT LI LU NL SE**

(56) References cited:
**DE-A-2 514 873**
**GB-A-1 133 800**
**US-A-4 006 218**
**US-A-4 075 353**
**US-A-4 126 681**

The file contains technical information
submitted after the application was filed and
not included in this specification

(73) Proprietor: **THE PROCTER & GAMBLE COMPANY**
**301 East Sixth Street**
**Cincinnati Ohio 45202 (US)**

(72) Inventor: **Wickett, Richard Randall**
**4034 Jennifer Dr.**
**Hamilton Ohio 45013 (US)**
Inventor: **Cooper, Eugene Rex**
**2424 Ambassador Dr.**
**Cincinnati Ohio 45231 (US)**
Inventor: **Loomans, Maurice Edward**
**5231 Jessup Rd.**
**Cincinnati Ohio 45239 (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to pharmaceutical compositions containing a penetrating vehicle which markedly enhances the delivery of the drug active across human skin.

Because of the accessibility and large area of the skin, it has long been considered a promising route for the administration of drugs, whether dermal, regional, or systemic effects are desired.

The advantages of the topical route of drug administration include: avoidance of the risks and inconvenience of parenteral treatment; avoidance of the variable absorption and metabolism associated with oral treatment; continuity of drug administration, permitting use of pharmacologically active agents with short biological half-lives; potential reduction of gastrointestinal irritation in systemic administration; and treatment of cutaneous manifestations of diseases usually treated systemically.

However, the impermeability of skin is well-known, serving as a barrier to ingress of pathogens and toxic chemicals, and egress of physiologic fluids. This impermeability is the result of normal physiologic changes in developing skin. A typical cell in the epidermis is formed in the basal layer. It typically takes approximately thirty days for a cell to migrate from the basal layer of the epidermis to sloughing off and discarding at the outer layers of the stratum corneum. As the cell migrates outward from the basal layer, it progressively keratinizes until it is relatively impermeable. The result is the stratum corneum, an extremely thin surface layer (10 micrometers) with substantial barrier properties. The cell envelopes of the cells in the stratum corneum tend to be mainly polar lipids, such as ceramides, sterols, and fatty acids while the cytoplasm of stratum corneum cells remains polar and aqueous. Despite the close packing of the cells, some 15% of the stratum corneum is intercellular and, generally, lipid based. It is generally recognized that over the very short term, penetration occurs through the hair follicles and the sebaceous apparatus; long-term penetration occurs across cells (nonpolar route). Poor penetration of many drugs across the epidermal lipid barrier has, until now, frustrated attempts to deliver clinically significant doses of many drugs by the topical route.

The present invention relates to the discovery that, by increasing the disorder of the lipid portion of the cell envelopes in the stratum corneum, the lipid packing of the envelope can be disrupted, thereby liquifying it and allowing lipid soluble drugs to pass through the stratum corneum. In particular, it has been discovered by differential scanning calorimetry that certain binary skin penetration enhancement systems eliminate the TM-2 peak associated with melting of cell envelope lipids.

The enhanced penetration provided by the present invention now makes the topical mode of treatment practical by percutaneously delivering clinically effective doses of active drug.

1,2-propanediol ("propylene glycol") and the $C_{10}$—$C_{14}$ alcohols have been used, separately, in cosmetic and pharmaceutical formulations. In particular, propylene glycol has been described in several articles in the literature as enhancing the penetration of certain pharmacologically active agents, such as the corticosteroids.

US—A—3,535,422 relates to stable benzoyl peroxide compositions containing organic emollients. The compositions include emollients selected from the $C_4$—$C_{20}$ aliphatic alcohols, $C_2$—$C_3$ glycols, $C_{12}$—$C_{20}$ fatty acids and their esters, and mixtures thereof.

US—A—4,070,462 describes topical steroid compositions containing 6% propylene glycol and 1% propylene glycol monostearate.

CA—A—1,072,009, describes topical antimicrobial compositions containing $C_5$—$C_{10}$ straight chain alcohols or $C_{17}$ branched chain alcohols in which the longest chain is $C_5$—$C_{10}$.

CA 92:153,181j; describes an indomethacin ointment containing 10% propylene glycol and 1.1% diisopropanolamine.

US—A—2,990,331 describes tetracycline compositions containing carboxylic acid alkylolamides.

H. Barnes, et al., *Br. J. Derm.* 93, 459 (1975) describe testing of fluocinonide and fluocinolone acetonide in a vehicle describes as fatty alcohol propylene glycol (FAPG).

P. J. W. Ayres, et al., *Br. J. Derm.*, 99, 307 (1978), report comparative skin penetration of cortisol from commercially available cortisol ointments.

Schaaf and Gross, *Dermatologica, 106*, 357 (1953) note that unsaturated fatty acids and $C_6$—$C_{14}$ saturated fatty acids are particularly active in provoking epidermal thickening.

J. Zatz, et al., *J. Pharm. Sci., 67*, 789 (1978) study the effect of formulation factors on penetration of hydrocortisone through mouse skin.

S. K. Chandrasekaran, et al., *J. Pharm. Sci., 67*, 1370 (1978) discuss the pharmacokinetics of drug permeation through human skin.

B. Idson, *Cosmetics & Toiletries, 95*, 59 (1980), states the factors affecting drug penetration and, consequently, in most cases effectiveness, are complex. He observes that the vehicle that provides ideal conditions for one drug may prove unsatisfactory for another. The author concludes that prediction is not simple and product stability must be assessed by human trials. The same article indicates that Synalar Cream, a topical corticosteroid preparation, contains sorbitan monooleate and propylene glycol.

M. M. Rieger, *Cosmetics & Toiletries, 94*, 32—37 (1979) and *95*, 26—38 (1980), provides reviews of current literature in the area of skin penetration.

J. A. Sands, et al., *The Pharmacological Effect of Lipids*, 76 (1978) report that fatty acids and fatty acid derivatives are agents which can "interact with lipids and membrane proteins" to inhibit reproduction of lipid containing bacteriophage in controlled studies. No mechanism or structure-function relationship is elucidated.

US—A—4,299,826 describes a composition for the treatment of acne by using diisopropyl sebacate as a penetration enhancer for an erythromycin derivative in combination with an alcohol.

US—A—2,990,331 describes the parenteral administration of tetracycline salts from a stable aqueous solution.

CA 79: 122,308 describes an electromagnetic study of n-alkyl ionic surfactants as aiding in human epidermis penetration.

According to the present invention, there is provided a composition for topical administration which includes:

(a) from 0.01% to 35% by weight of a pharmacological active selected from nonsteroidal anti-inflammatory agents, local anesthetics, antibiotic agents, and benzoyl peroxide;

(b) from 0 to 70% by weight of ethanol or 2-propanol; and

(c) from 0.01% to 99% by weight of a penetration-enhancing carrier;
characterized in that the penetration-enhancing carrier consists essentially of

(i) a diol compound selected from 1,2-ethanediol, 1,2-propanediol, 1,3-propanediol, 1,2-butanediol, 1,3-butanediol, 1,4-butanediol, 2,3-butanediol, 2-ethoxyethanol, 2,2-oxy-bis-ethanol and glycerol monoacetate; and

(ii) a cell envelope-disordering compound selected from methyl myristate, methyl laurate, ethyl laurate, ethyl myristate, myristyl acetate, lauryl acetate and mixtures thereof;

the ratio of cell envelope-disordering compound:diol compound being in the range of from 1:1 to 1:500 by weight.

Binary mixtures of any of the foregoing diol compounds and envelope disordering compounds, in a weight ratio of diol compound:cell envelope disordering compound of from 1:1 to 500:1, provide significant enhancement of penetration for a variety of nonpolar or lipophilic compounds, as well as a few compounds which appear to penetrate by both polar and nonpolar routes. A ratio (envelope-disordering compound:diol) of from 1:100 to 1:10 for the combination is preferred, and most preferably 1:50 to 1:10. The ratio most highly preferred is about 1:30 for the practice of this invention.

Nonsteroidal anti-inflammatory agents

Nonpolar or lipophilic compounds which are especially useful when delivered by the vehicles of this invention are the nonsteroidal anti-inflammatory agents, including salicylic acid, ibuprofen, sulindac, naproxen, ketoprofen, etofenamate, and indomethacin, and their pharmaceutically acceptable salts and esters.

Among the nonsteroidal anti-inflammatory agents, few have the safety and efficacy record accumulated by the salicylates. thus, salicylic acid and its common derivatives, such as aspirin, glycol salicylate, salicylamide, are particularly preferred embodiments of the nonsteroidal anti-inflammatory compositions herein. Another especially preferred nonsteroidal anti-inflammatory drug, particularly for patients with sensitivity to salicylates, is indomethacin, as well as its common salts and esters. A particularly preferred form of indomethacin is indomethacin methyl ester.

Local anesthetics

Another group of lipophilic pharmacologic actives whose penetration is significantly enhanced from the compositions of the present invention are the local anesthetics, including, without limitation, lidocaine, procaine, mepivacaine, bupivacaine, dibucaine, and tetracaine, as well as their pharmaceutically acceptable salts and esters.

Among the local anesthetics, few have the safety and efficacy record accumulated by lidocaine. Thus, lidocaine is an especially preferred pharmacological active for use in the local anesthetic compositions of this invention. However, depending upon particular sensitivities of individual patients, and the desired duration of local anesthetic effects, other local anesthetics may be preferable in individual instances.

Antibiotics

Another group of compounds having pharmacologic activity which is especially suitable for topical administration by the compositions provided herein are the antibiotics, including, without limitation, erthromycin, penicillin, clindamycin, tetracycline, and chloramphenicol, as well as their pharmaceutically acceptable salts and esters. Of these, erythromycin, clindamycin, and tetracycline are especially useful, and preferred, for the antibiotic treatment of acne. Another commonly employed topical antibacterial mixture is a combination of polymyxin B, bacitracin, and neomycin. This composition is also suitable for administration with enhanced penetration via the compositions of this invention.

In dealing with non-acneiform dermatologic infections, penicillin is the most universal drug of first choice. Accordingly, penicillin, which, when used herein, includes the synthetic penicillins, particularly the penicillinase-resistant varieties, also forms an especially preferred embodiment of the antibiotic compositions of this invention. Another agent which is especially preferred for systemic use, particularly in patients with sensitivity to the penicillins, is erythromycin, including its common pharmaceutical salts and esters. Examples would include erythromycin ethyl succinate, erythromycin lactobionate and erythromycin estolate. A third, particularly preferred composition, widely used in topical antibiotic ointments, is the combination of polymyxin B, bacitracin, and neomycin.

Benzoyl peroxide

Another material especially suitable for administration with the penetration enhancers of this invention is benzoyl peroxide.

Benzoyl peroxide is an item of commerce. It is preferably incorporated at levels from 0.5% to 25%, more preferably from 1% to 15% and more preferably from 2% to 10%. In general, when formulating compositions for topical administration of benzoyl peroxide, the more benzoyl peroxide the better, the limiting factor usually being the skin irritation which commonly accompanies benzoyl peroxide administration.

The compositions are typically prepared by thoroughly blending all of the components together in admixture, and milling, if necessary, to reduce all particles of benzoyl peroxide to impalpable size (typically less than 0.25 mm). The benzoyl peroxide should be of high purity, on the order of 97% to 100% pure, and in the form of a finely divided powder. The powder may be either wet or dry, but is preferably wet for ease of handling and safety. If wet benzoyl peroxide is used, it may be necessary to grind the crystals before admixture or to mill the composition after admixture and blending to reduce the crystals to impalpable size. Preferably, the milling or grinding operation is performed with cooling to prevent decomposition of the peroxide by localized friction.

The compositions of this invention are typically applied twice daily to the afflicted situs, or, when systemic effects are desired, to larger areas, depending upon the dose desired. A typical safe and effective usage rate is about 1 mg of the total composition/cm$^2$ skin to about 10 mg/cm$^2$ skin, per application, but this can vary with the use, the severity of the affliction, and the nature and concentration of pharmacological active, as well as the particular composition of the topical carrier being used. In particular, substantially higher application rates (up to 500 mg/cm$^2$) can be employed in conjunction with occlusive dressings.

The compositions can be applied qd, q12h, q8h, q6h, q4h, or on any other convenient treatment regimen. A q4h schedule is particularly preferred because it minimizes the amount of drug which is applied at one time, without requiring inordinately frequent applications or amounts of the composition which are too small to be applied conveniently.

By "topical administration" herein is meant directly laying on or spreading on epidermal tissue, especially outer skin.

By "safe and effective amount" of the compositions herein is meant a sufficient amount of the composition to alleviate or prevent the disease state being treated at a reasonable benefit/risk ratio attendant with any medical treatment. Within the scope of sound medical judgment, the amount of pharmaceutical active used will vary with the particular condition being treated, the severity of the condition, the duration of the treatment, the specific compound employed and its concentration, and like factors within the specific knowledge and expertise of the patient or the attending physician.

By "toxicologically or pharmaceutically acceptable" herein is meant ingredients which are suitable for use in contact with the tissues of humans and lower animals without undue toxicity, irritation, allergic response, and the like, commensurate with a reasonable benefit/risk ratio.

By the term "comprising" herein is meant that various other compatible drugs and medicaments, as well as inert ingredients and cosmetic vehicles, can be conjointly employed in the compositions and processes of this invention, as long as the critical binary penetration enhancement vehicle and pharmaceutical active are used in the manner disclosed herein. The term "comprising" thus encompasses and includes the more restrictive terms "consisting of" and "consisting essentially of" which characterize the use of the essential ingredients in the manner disclosed herein.

By "afflicted situs" herein is meant a localized area of inflammation or lesion, and the immediate surrounding area. By "acne" herein is meant common acne, acne vulgaris, in all forms, including papular pustular or cystic.

By "nonsteroidal anti-inflammatory drug" is meant, generally, those drugs which appear to act, at least in part, by inhibition of prostaglandin synthetase. The variety of compounds encompassed by this phrase is well known to those of skill in the art, and reference may be had to standard texts, including *Antiinflammatory Agents, Chemistry and Pharmacology* Vol. 1, R. A. Scherrer, et al., Eds., Academic Press, New York, 1974, the disclosures of which are hereby incorporated herein by reference, for detailed disclosures of chemical structures, syntheses, side effects, etc.

By "penetration-enhancing" herein is meant that the binary penetration enhancing carriers of this composition provide marked transepidermal delivery of an incorporated pharmacological active, when compared to other compositions at equal chemical potential. This latter aspect is important, since varying solubilities of drugs in different vehicles will necessarily effect their transport across skin. Thus, for example, if a drug is soluble in vehicle A to the extent of 24%, and in vehicle B to the extent of 4%, if the compositions were to be compared at equal percentage concentration, rather than equal chemical potential, the lower solubility carrier will show a misleading six-fold difference in transport over the more soluble vehicle. The simplest way of assuring equal chemical potential for evaluating penetration enhancement is to use saturated solutions or solutions of equal percentage of saturation of pharmacological active in the various vehicles.

By "local anesthetic" herein is meant drugs that block nerve conduction when applied locally to nerve tissue in appropriate concentrations. They act on any part of the nervous system and on every type of nerve fiber. Their action is reversible, their use being followed by complete recovery in nerve function with no evidence of structural damage to nerve fibers or cells.

By "antibiotic agent" herein is meant a chemical substance produced by microorganisms or a derivative of a chemical substance produced by microorganisms that has the capacity, in dilute solutions, to inhibit the growth of other microorganisms or destroy them.

Industrial applicability
Penetration-enhancing carriers

Ratios for combinations of envelope-disordering compound:diol in the present invention are from 1:1 to 1:500. Preferably a ratio of 1:10 to 1:100 of cell envelope-disordering compound to diol should be employed in the present invention. Most preferably 1:10 to 1:50 are used, with a ratio of 1:30 being most highly preferred in the instant invention.

The compositions herein typically contain from 0.05% to 50% of the envelope-disordering compounds. The envelope-disordering compounds are methyl myristate, methyl laurate, ethyl myristate, ethyl laurate, myristyl acetate, and lauryl acetate, with methyl myristate and methyl laurate being especially preferred. These esters are preferably present in the composition at a concentration of .05% to 5% by weight, most preferably 1% to 3% by weight.

In addition to those parameters well-known to one skilled in the art of the present invention, such as cost, availability, solvent properties, solute properties, and stability, the cell envelope-disordering compounds of the instant invention possess the ability to enhance integument penetration while exhibiting little dermal irritation. While some dermal irritation is acceptable in the practice of this invention, the cell envelope-disordering compounds show less irritation alone than the sum of the irritation caused by the remaining total composition absent the cell envelope-disordering compound. Of course, a cell envelope-disordering compound that causes no noticeable dermal irritation is most highly preferred.

The compositions also typically contain from 5% to 98% by weight of a diol, 1,2-propanediol (propylene glycol) and 2,2'-oxy-bis-ethanol (diethylene glycol) are the preferred diols. The most preferred diol is 1,2-propanediol. The diol component is preferably present at a concentration of from 10% to 50% by weight, most preferably from 20% to 30% by weight.

The compositions typically contain a cosmetically acceptable solvent such as ethanol or isopropanol. The solvent, if one is used, should preferably evaporate rapidly and completely to leave only the active components of the composition at the site of application.

It can be seen from the foregoing that the compositions of the present invention admit of considerable variation, so long as the critical components of active drug, diol compound, and envelope-disordering compounds are present.

In some cases, solubility of the envelope-disordering compounds imposes a limitation on formulation. More specifically, solubility of these compounds in typical compositions decreases with increasing chain length. In general, the envelope-disordering compounds are soluble at concentrations which are more than adequate to enhance penetration of the active drug, and, in general, high concentrations tend to cause skin irritation without a commensurate increase in penetration enhancement. Indeed, at very high levels, penetration enhancement begins to fall off, as the levels of auxiliary compound reduce the concentration of the equally necessary diol compound.

In cases where solubility of one or more components creates a problem in formulation, all of the components will generally be soluble in ethanol, and the use of ethanol as a co-solvent in formulation is especially recommended.

Other optional components can be added to the formulations of this invention in minor amounts to enhance their cosmetic acceptability. Such components include thickening agents, such as methyl cellulose, cross-linked carboxypolymethylene polymers, bentonite, gum tragacanth, gum karaya, and polyethylene glycols. Such thickening agents are generally employed at a level of from about 1% to about 10% of the composition. Cosmetic resins and film formers such as the Carboset carboxyvinyl polymers can also be present. Small amounts of pigments and opacifiers, such as zinc oxide, and fragrance materials such as perfumes can also be used. Such formula modifications are well within the skill of workers in the cosmetic and dermatological arts, and, by themselves, constitute no part of the present invention. Many additives which enhance cosmetic acceptability undesirably interfere with penetration enhancement. Therefore, the use of the foregoing, and other, optional ingredients is preferably avoided.

The following example illustrate the broad range of utility of the compositions of the present invention, while not intending to limitative thereof.

Skin penetration studies

In the Example, human skin (heat-separated abdominal epidermis, taken at autopsy) was placed in a standard Franz diffusion apparatus (Crown Glass Co., Somerville, N.J.) in a horizontal position between a lower, capped diffusion cell and an upper, open cell. A normal saline solution was added to the lower diffusion cell, abutting the subcutaneous side of the skin, and the test composition comprising a solution of active drug in the carrier at indicated formulation was added to the diffusion cell abutting the epidermal side of the skin.

This cell assembly was kept in a constant-temperature room at about 31°C. At appropriate intervals, each diffusion cell assembly was opened and the diffusate from the cell abutting the subcutaneous side of the skin was withdrawn.

Drug active in the diffusate was measured using standard analytical techniques.

Example I

The following data show the effectiveness of various fatty acid esters and fatty alcohol acetate as penetration aids for indomethacin.

| Vehicle | Dose | Relative penetration |
|---|---|---|
| 1% Indo[1]+30/70 PG[2]/EtOH to 100% | 25 µl/cm$^2$ | 1±30% |
| 1% Indo+30/70 Pg/EtOH+1% MA | | 5±20% |
| 1% Indo+3% lauryl acetate+30/70 PG/EtOH to 100% | | 5±30% |
| 1% Indo+3% ethyl laurate+30/70 PG/EtOH to 100% | | 6±50% |
| 1% Indo+3% ethyl myristate+30/70 PG/EtOH to 100% | | 4±40% |
| 1% Indo+3% myristyl acetate+30/70 PG/EtOH | | 5±30% |

[1]Indomethacin.
[2]PG: propylene glycol.

## Claims

1. a composition for topical administration which includes:

(a) from 0.01% to 35% by weight of a pharmacological active selected from nonsteroidal anti-inflammatory agents, local anesthetics, antibiotic agents, and benzoyl peroxide;

(b) from 0 to 70% by weight of ethanol or 2-propanol; and

(c) from 0.01% to 99% by weight of a penetration-enhancing carrier;

characterized in that the penetration-enhancing carrier consists essentially of

(i) a diol compound selected from 1,2-ethanediol, 1,2-propanediol, 1,3-propanediol, 1,2-butanediol, 1,3-butanediol, 1,4-butanediol, 2,3-butanediol, 2-ethoxyethanol, 2,2'-oxy-bis-ethanol and glycerol monoacetate; and

(ii) a cell envelope-disordering compound selected from methyl myristate, methyl laurate, ethyl laurate, ethyl myristate, myristyl acetate, lauryl acetate and mixtures thereof;

the ratio of cell envelope-disordering compound:diol compound being in the range of from 1:1 to 1:500 by weight.

2. A composition according to Claim 1 characterized in the pharmacological active is a nonsteroidal anti-inflammatory agent.

3. A composition according to Claim 2 characterized in that the nonsteroidal anti-inflammatory agent is selected from salicyclic acid, ibuprofen, sulindac, naproxen, ketoprofen, ethofenamate, and indomethacin and the pharmaceutically acceptable salts and esters thereof.

4. A composition according to Claim 3 characterized in that the nonsteroidal anti-inflammatory agent is selected from indomethacin, the methyl ester of indomethacin, and mixtures thereof.

5. A composition according to any one of Claims 1—4 characterized in that the diol compound is selected from 1,2-propanediol, 2,2'-oxy-bis-ethanol and mixtures thereof.

6. A composition according to any one of Claims 1—5 characterized in that the cell-envelope disordering compound is selected from methyl myristate, methyl laurate and mixtures thereof.

7. A composition according to any one of Claims 1—6 characterized in that the cell envelope-disordering compound:diol compound ratio is from 1:10 to 1:100.

8. A composition according to any one of Claims 1 or 5—7 characterized in that the pharmacological active is an antibiotic agent selected from erythromycin, penicillin, tetracycline, chloramphenicol and the pharmaceutically-acceptable salts and esters thereof.

9. A composition according to any one of Claims 1 or 5—7 characterized in that the pharmacological active is benzoyl peroxide.

10. A composition according to any one of Claims 1 to 5—7 characterized in that the pharmacological active is a local anesthetic selected from lidocaine, procaine, mepivacaine, bupivacaine, dibucaine, tetracaine and the pharmaceutically-acceptable salts and esters thereof.

## Patentansprüche

1. Zusammensetzung zur örtlichen Anwendung, enthaltend:

(a) 0,01 bis 35 Gew.-% eines pharmakologischen Wirkstoffs ausgewählt aus entzündungshemmenden Mitteln, die keine Steroide sind, örtlichen Betäubungsmitteln, antibiotischen Mitteln und Benzoylperoxid;

(b) 0 bis 70 Gew.-% Ethanol oder 2-Propanol; und

(c) 0,01 bis 99 Gew.-% eines die Tiefenwirkung verbessernden Trägers;
dadurch gekennzeichnet, daß der die Tiefenwirkung verbessernde Träger im wesentlichen aus

(i) einer Diolverbindung ausgewählt aus 1,2-Ethandiol, 1,2-Propandiol, 1,3-Propandiol, 1,2-Butandiol, 1,3-Butandiol, 1,4-Butandiol, 2,3-Butandiol, 2-Ethoxyethanol, 2,2'-Oxy-bis-ethanol und Glycerinmonoacetate; und
(ii) einer die Zellhülle störenden Verbindung ausgewählt aus Methylmyristat, Methyllaurat, Ethyllaurat, Ethylmyristat, Myristylacetat, Laurylacetat und Gemischen daraus;

besteht, wobei das Gewichtsverhältnis von die Zellhülle störender Verbind zu Diolverbindung im Bereich von 1:1 bis 1:500 liegt.

2. Zusammensetzung gemäß Anspruch 1, dadurch gekennzeichnet, daß der pharmakologische Wirkstoff ein entzündungshemmendes Mittel, das kein Steroid ist, bedeutet.

3. Zusammensetzung gemäß Anspruch 2, dadurch gekennzeichnet, daß das entzündungshemmende Mittel, welches kein Steroid ist, ausgewählt ist aus Salicylsäure, Ibuprofen, Sulindac, Naproxen, Ketoprofen, Ethofenamat und Indomethacin und deren pharmazeutisch verträglichen Salzen und Estern.

4. Zusammensetzung gemäß Anspruch 3, dadurch gekennzeichnet, daß das entzündungshemmende Mittel, welches kein Steroid ist, ausgewählt ist aus Indomethacin, dem Methylester von Indomethacin und Gemischen daraus.

5. Zusammensetzung gemäß einem der Ansprüche 1 bis 4, dadurch gekennzeichnet, daß die Diolverbindung ausgewählt ist aus 1,2-Propandiol, 2,2'-Oxy-bis-ethanol und Gemischen daraus.

6. Zusammensetzung gemäß einem der Ansprüche 1 bis 5, dadurch gekennzeichnet, daß die die Zellhülle störende Verbindung ausgewählt ist aus Methylmyristat, Methyllaurat und Gemischen daraus.

7. Zusammensetzung gemäß einem der Ansprüche 1 bis 6, dadurch gekennzeichnet, daß das Verhältnis von die Zellhülle störender Verbindung zu Diolverbindung 1:10 bis 1:100 beträgt.

8. Zusammensetzung gemäß einem der Ansprüche 1 oder 5 bis 7, dadurch gekennzeichnet, daß der pharmakologische Wirkstoff ein antibiotisches Mittel ausgewählt aus Erythromycin, Penicillin, Tetracyclin, Chloramphenicol und deren pharmazeutisch verträglichen Salzen und Estern ist.

9. Zusammensetzung gemäß einem der Ansprüche 1 oder 5 bis 7, dadurch gekennzeichnet, daß der pharmakologische Wirkstoff Benzoylperoxid ist.

10. Zusammensetzung gemäß einem der Ansprüche 1 oder 5 bis 7, dadurch gekennzeichnet, daß der pharmakologische Wirkstoff ein örtliches Betäubungsmittel ausgewählt aus Lidocain, Procain, Mepivacain, Bupivacain, Dibucain, Tetracain und deren pharmazeutisch verträglichen Salzen und Estern ist.

**Revendications**

1. Composition pour administration topique qui comprend:

a) 0,01% à 35% en poids d'un agent ayant une activité parmacologique choisi parmi les anti-inflammatoires non stéroïdiens, les anesthésiques locaux, les antibiotiques et le peroxyde de benzoyle;

b) 0 à 70% en poids d'éthanol ou de 2-propanol; et

c) 0,01% à 99% en poids d'un excipient renforçant la pénétration;
caractérisée en ce que l'excipient renforçant la pénétration est constitué essentiellement de

(i) un diol choisi parmi le 1,2-éthanediol, le 1,2-propanediol, le 1,3-propanediol, le 1,2-butanediol, le 1,3-butanediol, le 1,4-butanediol, le 2,3-butanediol, le 2-éthoxyéthanol, le 2,2'-oxy-bis-éthanol et le monoacétate de glycérol; et
(ii) un composé désorganisant les enveloppes celluliares choisi parmi le myristate de méthyle, le laurate de méthyle, le laurate d'éthyle, le myristate d'éthyle, l'acétate de myristyle, l'acétate de lauryle et les mélanges de ceux-ci;

le rapport de la quantité de composé désorganisant les enveloppes cellulaires à celle du diol étant compris entre 1:1 et 1:500 en poids.

2. Composition selon la revendication 1 caractérisée en ce que l'agent ayant une activité pharmacologique est un anti-inflammatoire non stéroïdien.

3. Composition selon la revendication 2 caractérisée en ce que l'agent anti-inflammatoire non stéroïdien est chois parmi l'acide salicylique, l'ibuprofene, le sulindac, le naproxène, le ceto-profène, l'éthofénamate, et l'indométhacine et les sels et esters pharmacetiquement acceptables de ceux-ci.

4. Composition selon la revendication 3, caractérisée en ce que l'anti-inflammatoire non séroïdien est choisi parmi l'indométhacine, l'ester méthylique de l'indométhacine et les mélanges de ceux-ci.

5. Composition selon l'une quelconque des revendications 1 à 4, caractérisée en ce que le diol est choisi parmi le 1,2-propanediol, le 2,2'-oxy-bis-éthanol et les mélanges de ceux-ci.

6. Composition selon l'une quelconque des revendications 1 à 5, caractérisée en ce que le composé désorganisant les enveloppes cellulaires est choisi parmi le myristate de méthyle, le laurate de méthyle et les mélanges de ceux-ci.

7. Composition selon l'une quelconque des revendications 1 à 6, caractérisée en ce que le rapport des quantités de composé désorganisant les enveloppes cellulaires au diol est compris entre 1:10 et 1:100.

8. Composition selon l'une quelconque des revendications 1 ou 5 à 7, caractérisée en ce que l'agent ayant une activité pharmacologique est un antibiotique choisi parmi les éthromycine, pénicilline, tétracycline, chloramphénicol et les sels et esters pharmacetiquement acceptables de ceux-ci.

9. Composition selon l'une quelconque des revendications 1 ou 5 à 7 caractérisée en ce que l'agent ayant une activité pharmacologique est le peroxyde de benzoyle.

10. Composition selon l'une quelconque des revendications 1 ou 5 à 7, caractérisée en ce que l'agent ayant une activité pharmacologique est un anesthésique local choisi parmi la lidocaïne, la procaïne, la mépivacaïne, la bupivacaïne, la dibucaïne, la tétracaïne et les sels et esters pharmaceutiquement acceptables de celles-ci.